# EUROPEAN PATENT APPLICATION

(11) **EP 4 159 111 A1**
(43) Date of publication of application: **05.04.2023**
(21) Application number: 21200278.6
(22) Date of filing: 30.09.2021
(51) Int. Cl.: A61B 5/00, A61B 5/06, A61N 5/06

(54) **SKIN TREATMENT DEVICE WITH AN IMAGING UNIT, AND METHOD THEREOF**

(71) Applicant: Koninklijke Philips N.V., 5656 AG Eindhoven (NL)
(72) Inventor: VAN ABEELEN, Frank Anton, Eindhoven (NL); VERHAGEN, Rieko, Eindhoven (NL); BOAMFA, Marius Iosif, Eindhoven (NL); THUMMA, Kiran Kumar, Eindhoven (NL)
(74) Representative: Philips Intellectual Property & Standards

(57) **Abstract**

Provided is a method and device thereof, for determining whether to perform skin treatment by a device. The method comprises obtaining an image frame of a skin area to be treated, sensing a displacement of the device during a processing period of the captured image frame and determining whether to perform the skin treatment based on the sensed displacement. The device comprises an image capturing unit configured to capture an image frame of a skin area to be treated, a motion/position sensor configured to sense a displacement of the device on the skin area, and a control means configured to process the captured image frame during a processing period, receive, from the motion sensor, a displacement of the device during the processing period, and determine whether to perform treatment based on the received displacement.

## Description

### FIELD OF THE INVENTION

The present application relates to a device for skin or hair treatment and method thereof, and specifically, to a device comprising an imaging or image capturing unit, and method thereof.

### BACKGROUND OF THE INVENTION

Skin treatment devices with imaging units are known. Skin treatment devices which involve phototherapy or photo-epilation are also known. In some cases, the treated skin area is recorded by imaging to avoid cases that a subsequent treatment step is not carried out on the same skin area. This may also be implemented by displacement sensors, which detect a current position relative to the position of the treated area. Repetitive treatment of a skin area, either by overlapping of treated areas or by unintentional repeated application of treatment on a previously treated skin area could cause injury to the skin. The recording of treated areas is even more desirable when the treatment is carried out using light, e.g., in applications such as intense pulsed light (IPL). In other cases, the skin to be treated is recorded before treatment, and analyzed for presence of blemishes or tattoos, in order to selectively treat such areas using the correct light intensity without treating the surrounding skin. The below details relate to devices which use light, amongst other forms of energy, for non-therapeutic skin treatment.

### SUMMARY OF THE INVENTION

While using cosmetic skin treatment devices, especially photo-epilation devices, it is an object to obtain information of skin to be treated, before performing the treatment. In an IPL device, e.g., it is generally recommended to avoid treating skin area with blemishes or tattoos for safe use of the device.

Such information can be obtained by means of imaging elements incorporated in the device. However, imaging units like camera involve a certain latency while capturing and processing image data. In other words, period T, starting a time instant when an image frame is captured and an instant the captured image frame is processed (including data readout and data transfer), is greater than zero. Further, the higher the image resolution, the higher the latency. It is desirable to goal to decrease this latency or delay, however, for reasons involving added cost of the device and ease of manufacturing, this is not always achieved.

A problem exists that once an image of a skin area is captured by the imaging unit, the user may intentionally or non-intentionally move the skin treatment device from the skin area. In this case, the processed image will no longer reflect the features of the skin under that device at that particular moment. This is not desirable, as the treatment would be executed on a non-analyzed part of the skin.

An object of the present invention to provide a method and a device thereof, which takes into account the delay in processing images to perform effective skin treatment.

According to an aspect of the invention, a device for skin treatment is provided. The device comprises an image capturing unit which is configured to capture an image frame of a skin area to be treated, a motion or position sensor configured to sense a displacement of the device on the skin area, and a control means configured to process the captured image frame during a processing period, receive, from the motion sensor, a displacement of the device during the processing period, and determine whether to perform treatment based on the received displacement. A method for determining whether to perform skin treatment by a device is further provided. The method is computer-implemented. The method comprises the steps of obtaining an image frame of a skin area to be treated, sensing a displacement of the device during a processing period of the captured image frame and determining whether to perform the skin treatment based on the sensed displacement.

According to another aspect of the invention, the control means is further configured to enable a treatment source when the sensed displacement is zero and/or disable the treatment source when the sensed displacement is non-zero. The corresponding method comprises enabling the treatment when the sensed displacement is zero and disabling the treatment when the sensed displacement is non-zero.

According to yet another aspect of the invention, the device comprises a treatment source. In other words, the device is self-contained, including the image capturing unit, the motion sensor, the control means. It can determine whether or not to perform the treatment, as well as perform it.

According to yet another aspect of the invention, the control means is further configured to enable the treatment source upon determining that the imaged treatment area is suitable for skin treatment. The corresponding method comprises the step of enabling the treatment source upon determining that the imaged treatment area is suitable for skin treatment.

According to yet another aspect of the invention, the device further comprises a user interface unit. The control means is configured to control the image capturing unit to capture the image frame, upon detecting a user input to the user interface unit. The corresponding method includes controlling the image capturing unit to capture the image frame, upon detecting a user input to the user interface unit.

According to yet another aspect of the invention, the motion sensor is further configured to detect an initial position of the device with respect to the skin area, and the control means is configured to control the image capturing unit to capture the image frame based on the detection. The corresponding method comprises the step of detecting an initial position of the device with respect to the skin area by the motion sensor, and controlling the image capturing unit to capture the image frame based on this detection.

According to yet another aspect of the invention, the motion or position sensor comprises a skin contact sensor. In other words, the initial position is detected via contact with the skin area. Once contact is registered, the image capturing unit is enabled.

According to yet another aspect of the invention, the control means determines whether to perform treatment based on whether contact remains established with the imaged skin area during the processing period. The corresponding method comprises determining whether to perform treatment based on whether contact remains established with the imaged skin area during the processing period.

According to yet another aspect of the invention, the control means is further configured to control the image capturing unit to capture a new image frame during the processing period based on the received displacement. The method includes controlling the image capturing unit to capture a new image frame during the processing period based on the received displacement.

According to yet another aspect of the invention, the processing period is 10-1000 milliseconds, preferably 100-500 milliseconds, more preferably 20-50 milliseconds.

According to yet another aspect of the invention, the treatment source is a light-based source, such as a light emitting diode, a laser or a halogen lamp.

According to yet another aspect of the invention, a computer program product is provided, which comprises a computer readable medium having computer readable code embodied therein, the computer readable code being configured such that, on execution by a suitable computer or processing unit, the computer or processing unit (control means) is caused to perform the method described above, and the details listed in the below sections.

These and other aspects, and further advantages, will be apparent from and elucidated with reference to the embodiment(s) described herein.

### BRIEF DESCRIPTION OF THE DRAWINGS

Fig. 1 shows a block diagram of a device 1 for skin treatment according to an embodiment of the invention.
Fig. 2 shows another block diagram of a system comprising device 1 and a skin treatment device 10 for skin treatment according to an embodiment of the invention.
Fig. 3 shows a skin treatment device 10 according to an embodiment of the invention.
Fig. 4 shows a general method carried out by device 1 or 10 according to an exemplary embodiment of the invention.
Fig. 5 shows a method for detecting displacement of device 1 or skin treatment device 10 while processing the image frame and performing a control action based thereupon.
Fig. 6 shows a method of determining whether to perform skin treatment using device 1 or skin treatment device 10 according to an exemplary embodiment of the invention.

### DETAILED DESCRIPTION OF THE EMBODIMENTS

The matters exemplified in this description are provided to assist in a comprehensive understanding of various exemplary embodiments of the present invention disclosed with reference to the accompanying figures.

Accordingly, those of ordinary skill in the art will recognize that various changes and modifications of the exemplary figures/embodiments described herein can be made without departing from the scope of the claimed invention. In particular, combinations of specific features of various aspects of the invention may be made. An aspect or embodiment of the invention may be further advantageously enhanced by adding a feature that was described in relation to another aspect or embodiment of the invention.

Further, the functionality associated with any particular means may be centralized or distributed, whether locally or remotely. A computer program may be stored/distributed on a suitable medium, such as an optical storage medium or a solid-state medium supplied together with or as part of other hardware, but may also be distributed in other forms, such as via the Internet or other wired or wireless telecommunication systems. It may be advantageous to set forth that the terms "include" and "comprise," as well as derivatives thereof, mean inclusion without limitation. In addition, reference to an element by the indefinite article "a" or "an" does not exclude the possibility that more than one of the element is present, unless the context clearly requires that there be one and only one of the elements. The indefinite article "a" or "an" thus usually means "at least one".

The expression "at least one of A, B and C" means "A, B, and/or C", and that it suffices if e.g. only B is present. Any reference signs in the claims should not be construed as limiting the scope.

Terms such as, for example, "processing," "computing," "calculating," "determining," "establishing", "analyzing", "checking", or the like, used herein, may refer to, in a non-limiting manner, operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium (e.g., a memory) that may store instructions to perform operations and/or processes. The terms "plurality" and "a plurality" as used herein may include, for example, "multiple" or "two or more". The terms "plurality" or "a plurality" may be used throughout the specification to describe two or more components, devices, elements, units, parameters, or the like. Unless explicitly stated, the method embodiments described herein are not constrained to a particular order or sequence. Additionally, unless otherwise specified, some of the described method embodiments or elements thereof can occur or be performed simultaneously, at the same point in time, or concurrently.

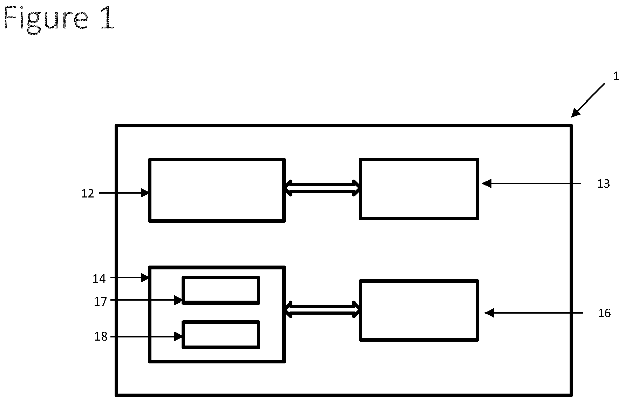

Fig. 1 shows a block diagram of a device 1 for skin treatment according to an embodiment of the invention. Device 1 is configured to determine whether to perform skin treatment as described below. Device 1 is suitable for skin treatment, as will be clear from below. In this embodiment, it is not considered necessary for device 1 to be able to perform treatment. It makes the decision to perform treatment.

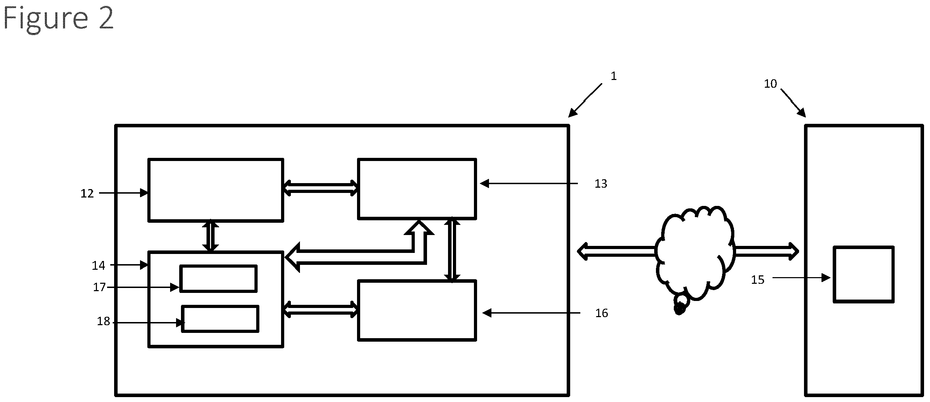

Fig. 2 shows another block diagram where device 1 is shown coupled with a skin treatment device 10. The system can then be used to perform skin treatment.

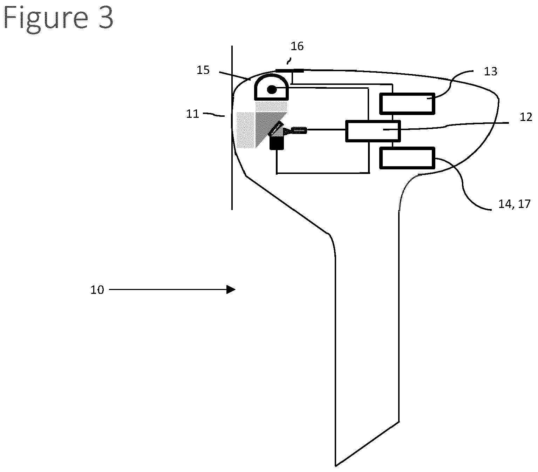

In Fig. 3, the device 1, or the components comprised therein, are part of the skin treatment device 10. Skin treatment device 10 may be an IPL device.

Skin treatment device 10 includes a treatment head 11. Treatment head 11 is configured to be placed on a skin surface when treatment is being applied to that skin surface (skin area). In an embodiment, treatment head 11 may be detachable and re-attachable to a remaining portion of skin treatment device 10.

In Fig. 1 embodiment, device 1 includes at least an image capturing unit 12, a control means 13 and a motion/position sensor 14.

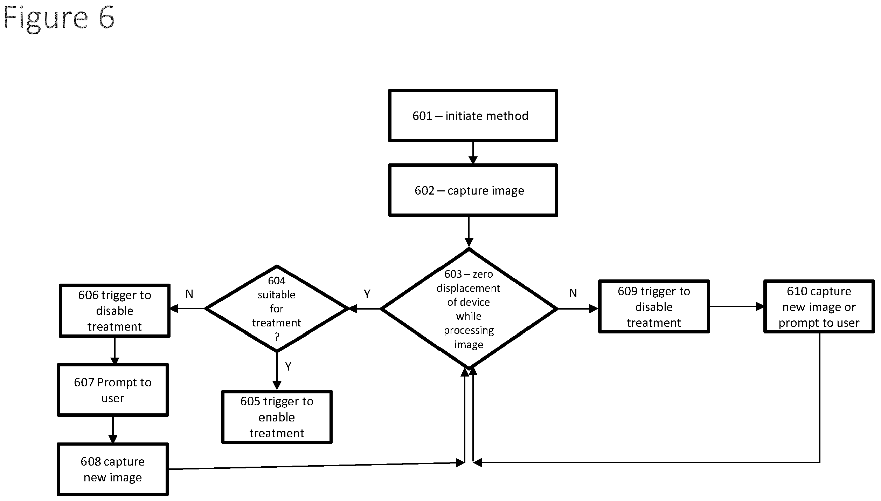

In an embodiment as shown in Fig. 3, the device 1 is incorporated in a light-based skin treatment device 10. An example of implementing the image capturing unit 12 in skin treatment device 10 is disclosed in Fig. 2, Euro-PCT publication WO2021122703 or Fig. 6, EP application number 21180439.8. As mentioned below, the invention is not limited to light-based treatment.

In another embodiment such as in Fig. 1 or 2, the image capturing unit 12 may be a camera of a mobile user device 1 which may be removably attached to the skin treatment device 10 at a suitable location on its housing (e.g., via a mechanical receptacle - as disclosed in Fig. 2, EP application number 20170690.0 or PCT/EP2021/059613). In this case, it is known to the skilled person how to structurally modify the skin treatment device and/or device 1 for device 1 to be able to control the application of skin treatment. Device 1 and skin treatment device 10 may be connected to each other via a wireless communication system, as shown in Fig. 2.

In a non-limiting manner, in the skin treatment device 10, the at least one image capturing unit 12 may be comprised in a head portion of device 10. This is shown in Fig. 3.

The image capturing unit 12 is configured to capture an image frame of a skin area to be treated. In an embodiment, the image capturing unit 12 captures an image frame upon user-initiation i.e., upon registering user input via operation of user interface unit 16 of device 1 or 10. In another embodiment, the image capturing unit 12 captures an image frame based on skin contact measurements. Details of how this is implemented are given in the below parts of the description. The skin area thus captured by the image frame is termed the imaged skin area herein. As mentioned, such image capture is targeted to study or analyze the skin area before the treatment is carried out. If there exists an area with a mole or other skin lesion on the skin, it is desirable to not carry out treatment on such area.

The control means 13 receives the captured image frame from the image capturing unit 12 and processes the captured image frame.

The control means 13 may be internal to the skin treatment device 10 or located in an external device, in which case, the skin treatment device 10 receives the processed information from the external control means. This can be implemented via e.g., wireless communication technology such as Bluetooth, NFC, WiFi etc., the options being known to the skilled person. Fig. 2 shows an example of a system comprising device 1 and skin treatment device 10 coupled to each other via a wireless communication system.

In an embodiment, the image capturing unit 12 may process the captured image. In yet another embodiment, the image may be jointly processed by the image capturing unit 12 and the control means 13.

Depending on the resolution of the image (the image capturing unit 13) and the processing power of the control means 13, there is a certain processing period required to process the captured image. This period includes time required to transfer/receive the captured image data, process the data and/or output the processed data.

The image processing period may be of the order of a few milliseconds, e.g., 20 milliseconds, to hundreds of milliseconds.

In an embodiment, as shown in Fig. 1 or 2, the motion sensor 14 is external to the skin treatment device 10 and comprised in device 1. Motion sensor 14 then senses displacement of device 1 on the skin area. Since device 1 is coupled to skin treatment device 10, the motion sensor 14 can further process the displacement of device 1 to correlate to displacement of skin treatment device 10 on the skin area. This is especially the case when device 1 is physically attached to device 10. In an embodiment, the image capturing unit 12, the motion sensor 14 and control means 13 may be controlled via a software application stored in a memory of device 1. Device 1 and the method thereof may then be controlled, amongst others, via user input through the software application.

In another embodiment, as shown in Fig. 3, the motion sensor 14 is internal to the skin treatment device 10. Motion sensor 14 then senses displacement of skin treatment device 1 on the skin area.

Device 1 or skin treatment device 10 may include additional circuitry 18, such as additional sensors or components such as transceivers, for enabling communication in a wired or wireless manner.

The motion sensor 14 in device 1 is communicatively coupled with control means 13. As mentioned, control means 13 can be located in device 1 or skin treatment device 10. It is not necessary that the image capturing unit 12, control means 13 and motion sensor 14 be located in one device.

In the embodiment of Fig. 3, motion sensor 14 includes one or more displacement sensing devices that are configured to sense a displacement of treatment head 11 on a skin surface. It is also possible for the motion sensor 14 to use another reference instead or in addition to the treatment head 11 in the skin treatment device 10 to measure displacement.

In an embodiment, the motion sensor 14 is a mechanical displacement sensor.

In another embodiment, the motion sensor 14 is an optical sensor, e.g., a camera, using sequential images.

In an embodiment, the image capturing unit 12 may also function as the optical motion sensor 14.

In an embodiment of Fig. 1, motion sensor 14 may be implemented using an external camera, such as that in a mobile user device (e.g., a smartphone). The method may utilize a motion sensor 14 of a mobile device which functions as the image capturing unit 12. The control means 13 analyses the images to detect whether a skin feature at one reference position in a first image frame has moved from the position to another in second image frame.

The control means is configured to receive from motion sensor 14 information on a displacement of device 1 and/or skin treatment device 10.

It is an object of the present invention to ensure that the imaged skin area corresponds to the skin area which is treated in a subsequent step. For sake of conciseness, the below discussion focuses on the embodiment where the image capturing unit 12, the control means 13 and motion sensor 14 are part of the skin treatment device 10, as shown in the embodiment of Fig. 3.

The motion sensor 14 senses displacement of the treatment head 11 during the image processing period of the captured image frame. The image processing period can be a predetermined/pre-stored value (corresponding to the device parameters) or be variable (depending on different image resolutions, programming instructions), and defines the time the device 10(or 1) takes to process a certain image frame.

In an embodiment the motion sensor 14 senses displacement of the treatment head 11 relative to the imaged skin area during the image processing period. The control means 13 is configured to extract position information of the imaged skin area from the captured image frame and/or a skin feature therein and obtain displacement of the imaged skin area and/or skin feature by analyzing a subsequent image frame captured by the image capturing unit 12 or the optical motion sensor 14 or by obtaining position coordinates from a mechanical motion sensor.

Based on the sensed displacement, the control means 13 controls a function of a treatment source 15 of the skin treatment device 10.

As mentioned, in a practical implementation, the device 1 comprising the image capturing unit 12, the control means 13 and the motion sensor 14 may be part of a mobile user device or smartphone, which is suitable for controlling the application of skin treatment by communicating with a treatment device (and thereby controlling the treatment device).

The treatment source 15 may be located in the treatment head 11 of the skin treatment device 10. For example, treatment source 15 may include a radiation source (e.g., of visible, ultraviolet, infrared, or radiofrequency radiation), an electromagnetic device, a heat source, a mechanical device (e.g., vibrator, mechanical massager, shaver, or other mechanical device), or another skin treatment device. The example shown in Fig. 1 relates to a skin treatment device 10 in which the treatment source 15 is a radiation source. The treatment source 11 is not limited to being situated in the treatment head 11 but can be disposed elsewhere in the skin treatment device 10, as long as there are other means to transmit the emitted energy (light, heat, sound, vibration, rotation etc.) to the skin to carry out the treatment. Operation of treatment source 15 is controlled by control means 13. The control means 13 may control operation of treatment source 15, in other words, the application of skin treatment, in accordance with at least one of user input via operation of user interface unit 16, sensed information from motion sensor 14, characteristics of the captured image frame by the image capturing unit 12, and programmed instructions.

### Sensed information from motion sensor 14

The control means 13 is configured to receive information on displacement of the skin treatment device 10 and/or treatment head 11 (or device 1, as mentioned above) during the processing period of the captured image frame, or while processing the image frame. The displacement may be measured relative to a position of the last imaged skin area.

The control means 13 determines whether to perform the treatment based on the sensed displacement. For example, it could enable or disable the treatment source 15 based on the measured displacement.

If the displacement during the image processing period is zero, the treatment source 13 is enabled. The treatment source 13 can be enabled by sending a trigger signal to a user interface unit 16, to indicate to the user to push a button on the skin treatment device 10 or be automatically enabled by the control means 13. It is determined that the skin treatment device 10 has not moved from the last imaged skin area, or since an instant when a previous image was captured [T0, P0→T1, P0]. Here, P0 is the set of position coordinates of the imaged skin area, registered at time T0 when the image of the skin area was captured by the image capturing unit 12. At time T1, when the image has been processed by the control means 13, the sensed position is still P0, meaning the device has been stationary during the image processing period T1-T0.

If the displacement during the image processing period is non-zero, the treatment source 13 is disabled. It is determined that the skin treatment device 10 has moved from the last imaged skin area, or since an instant when a previous image was captured [T0, P0→T1, PI]. Here, P0 is the set of position coordinates of the imaged skin area, registered at time T0 when the image of the skin area was captured by the image capturing unit 12. At time T1 when the image has been processed by the control means 13, the sensed position coordinates correspond to PI. PI no longer reflects the position of the skin area imaged or captured at time T0 by the image capturing unit 12.

The control means 13 may determine displacement instantaneously during the processing period, i.e. while processing the image frame, or after an estimated processing period T1-T0, the value of which may be pre-stored in the memory of the device. The value may be defined based on factors like image resolution, processing power of control circuitry etc.

In an embodiment, if the control means 13 detects that the displacement is non-zero when the image has been processed at time T1 or after the pre-defined processing period, it transmits a trigger signal to the image capturing unit 12 to capture a further (a second e.g.) image frame, the image frame being that of the skin area at the new position PI.

In an embodiment, if the control means 13 detects that the displacement is non-zero at an intermediate instant Tint during the image processing period T1-T0, it already transmits a trigger signal to the image capturing unit 12 to capture the further image frame, the image frame being that of the skin area at the new position at Tint, without adding a delay otherwise needed to process the previously captured image frame. The previously captured image frame may be deleted from a memory of the device or stored therein for future use.

As a result of the check during the processing period, it is possible for device 1 to determine whether there has been a displacement of the device since the last captured skin image and effect the treatment only if the imaged skin is the same as the treated skin (or the skin which would be treated).

### User input via operation of user interface unit 16

In an embodiment, the control means 13 is configured to send a trigger signal to the image capturing unit 12 when a user inputs a command via operation of user interface unit 16.

In an embodiment, the control means 13 is configured to detect whether the displacement is intentionally triggered by the user using user interface unit 16, e.g., by checking whether the displacement exceeds a predetermined threshold. The control means 13 may check whether the sensed displacement after image processing, at time T1, or at any time instant Tint during the image processing period.

### Characteristics of the captured image frame by the image capturing unit 12

It is another object of the invention to not perform treatment on areas which are unsuitable for treatment, such that those having skin irregularities including moles and tattoos.

In this regard, the control means 13 is configured to check whether the imaged skin area is suitable for treatment. It checks for the presence of skin characteristics which indicate unsuitability of the targeted skin area.

The control means 13 enables the treatment source (treatment pulse) upon determining that the imaged area is suitable for treatment. When the skin treatment device 10 is an IPL device, the treatment pulse is a light pulse.

The control means 13 transmits a signal to the user interface unit 16 to indicate to the user a change of treatment area, upon determining that the imaged skin area is unsuitable for skin treatment.

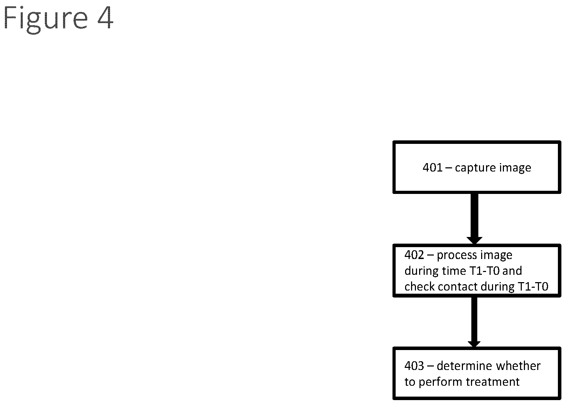

Fig. 4 shows a general method carried out by device 1 or 10, according to an exemplary embodiment of the invention.

Step 401 comprises obtaining an image frame of the skin area to be treated. The image is obtained either via manual trigger or automatically by detecting a mode of initiation such as contact or proximity detection. The start position is registered by the device.

Step 402 comprises sensing a displacement of the device during a processing period of the captured image frame. Displacement is calculated relative to the start position.

Step 403 comprises determining whether to perform the skin treatment based on the sensed displacement.

Details of this method are listed above, as steps performed by device 1 or 10.

As with the device functionalities, any of the above-mentioned steps carried out by the device may be combined in the method.

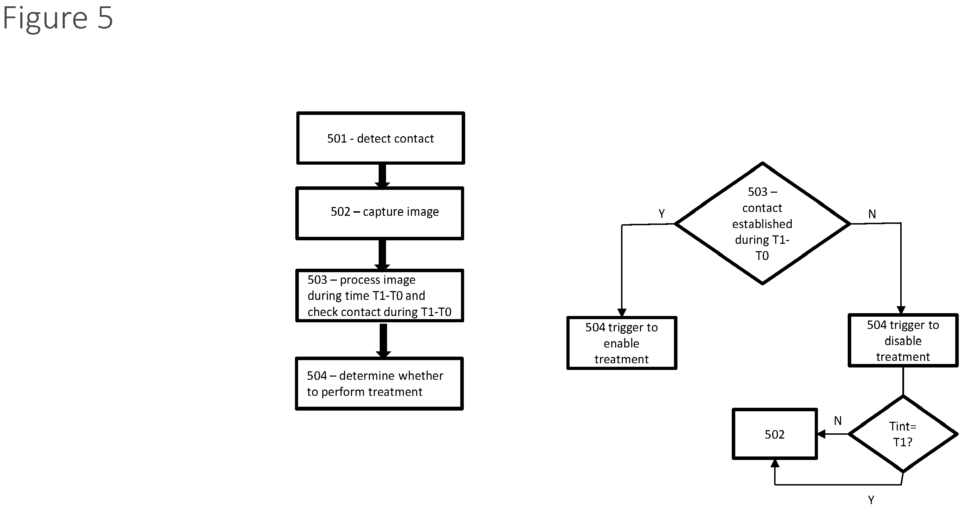

Fig. 5 relates to a method for detecting displacement of device 1 or skin treatment device 10 while processing the image frame and performing a control action based thereupon. In this specific embodiment, the motion sensor 14 is a skin contact sensor 17, i.e., device 1 further includes a skin contact sensor. It is well-known to the skilled person that any motion detection means may be implemented in the present invention.

The contact sensor may be optical or image-based (e.g., using a camera, and obtaining a difference in images in a contact vs no-contact mode) or a capacitance sensor which measures a change in capacitance upon registering contact of e.g. the treatment head 11 with skin. The skin contact sensor can alternately be ultrasound or a proximity sensor.

It is understood that the motion sensor is faster than the latency of the image capturing unit 12 (the time required to process the image). Depending on the image resolution that is acquired for processing, the latency might be in the range of tens of milliseconds or even longer. Therefore, it is preferred that the motion sensor offers a response time below a millisecond, ideally even faster.

In step 501, a step of detecting initial skin contact of a treatment head is performed by the skin contact sensor. The control means processes information obtained by the skin contact sensor 17 to determine contact with the skin area. The skin area where contact is registered is a start/initial position/area, relative to which motion is detected. In other words, the method utilizes information from a motion sensor (here, skin contact sensor) to initiate the treatment control.

In step 502, a step of capturing an image frame is performed by the image capturing unit 12.

In step 503, a step of processing the image frame is initiated by the control means 13. During this step, the control means continues to detect contact with skin via information obtained from the skin contact sensor 17 during the processing period.

In step 504, a step of controlling an application of skin treatment, or determining whether to perform the treatment, is performed by control means 13. For example, if contact with the skin is determined to be lost during the processing period (at any time instant while processing the image frame), the control means 13 determines that device 1 has displaced while processing the image frame, and that the skin area to be treated no longer corresponds to the imaged area. It sends a trigger signal to a treatment source 15 to disable it. As mentioned above, treatment source 15 can be located external to device 1. In addition, it may transmit a trigger signal to the image capturing unit 12 to obtain a second image frame at the new position where the device has registered contact and goes back to step 502. The second frame may be captured even during processing period T (Tint<T1), to avoid delay in device operation. This is especially useful when a predetermined processing period T1-T0 based on factors like image resolution, processing parameters etc. is pre-stored in the device, and the time instant when the displacement is detected is less than the predetermined value T1-T0. The method may further check whether the movement was user-initiated, and if not, display a warning to the user via the user interface unit 16 for motion control.

If at T1, it is determined that the skin contact sensor 17 registered contact throughout period T1-T0, then control means 13 sends a trigger signal to a treatment source 15 to enable it at the imaged skin area.

The above embodiments can be combined or be separately implemented in the present invention.

Fig. 6 shows a method of performing skin treatment using device 1 or skin treatment device 10 in an embodiment which combines at least some of the above specific steps.

In step 601, a method of initiating skin treatment is performed by control means 13. As mentioned above, the method can be initiated by the user via the user interface unit 16 and/or automatically by device 1 by registering contact with a certain skin area by the skin contact sensor 17 (which is an indication of a start position, as acquired by the motion sensor, as sufficiently clear from Fig. 3 embodiment).

In step 602, the method captures an image frame of the skin area to be treated.

In step 603, the image frame is processed. Simultaneous to processing the image, the method checks for a displacement of device 1 and/or 10. Based on this check, the method controls application of treatment to the imaged skin area. The control can be performed by sending a trigger signal to a treatment source to enable/disable it.

If there is zero displacement, i.e. if the device has not moved from the imaged skin area, in step 604, to ensure safety while performing skin treatment, it is determined whether the skin area is suited to treatment, e.g. by identifying certain skin characteristics in the image frame. If it is determined that the skin area is suited to treatment, the method enables the treatment source in step 605. Otherwise, if the skin area is not suited, the treatment source is disabled in step 606. In step 607, the user may be prompted to locate device 1 or 10 at a new skin area by a feedback signal transmitted by control means 13 via the user interface unit 16. The method captures a new image at the new skin area in step 608, and repeats the method at step 603.

If a non-zero displacement is determined, the treatment source (and hence the treatment) is disabled in step 609. The method may further calculate an extent of the displacement to check whether the displacement was intentional. If not, a message may be displayed to the user. In step 610, in an embodiment where capturing an image frame is initiated by registering contact with the skin contact sensor in step 601, the method may automatically capture a new image frame at the new skin area (after displacement), and the method is repeated from step 603. When the method is initiated by the user in step 601, a feedback may be signaled to the user via the user interface unit 16 to capture the new image frame. Method step 601 may be resumed for a next treatment session.

It is thus understood that a device according to the present invention can function according to any or a combination of the mentioned methods.

The present invention thus not only provides a position-triggered image capture but also a manner of treatment determination compensating for processing constraints of the image unit which captures the position of the device.

The control means 13 can be implemented in numerous ways, with software and/or hardware, to perform the various functions described herein. The control means 13 may comprise one or more microprocessors or digital signal processors (DSPs) that may be programmed using software or computer program code to perform the required functions and/or to control components of the control means 13 to effect the required functions. The control means 13 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The control means 13 can comprise or be associated with a memory. The memory can store data, information and/or signals (including image(s)) for use by the control means 13 in controlling the operation of the device 1 or 10 and/or in executing or performing the methods described herein. In some implementations the memory stores computer-readable code that can be executed by the control means 13 so that the control means 13 performs one or more functions, including the methods described herein. In particular embodiments, the program code can be in the form of an application for a smart phone, tablet, laptop, computer or server. The memory can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The user interface unit 16 may comprise transceivers which enable a data connection to and/or data exchange with other devices, including any one or more of servers, databases, user devices, and sensors. It can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). It may further comprise ciruitry to control any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

It will be appreciated that a practical implementation of device 1 or 10 may include additional components to those shown in the figures.

The dimensions and values disclosed herein are not to be understood as being strictly limited to the exact numerical values recited. Instead, unless otherwise specified, each such dimension is intended to mean both the recited value and a functionally equivalent approximation surrounding that value. Also, unless otherwise specified, the dimensions mentioned herein are measured using common laboratory measurement techniques known to the skilled person.

While the present disclosure has been described with the above-described exemplary embodiments, various changes and modifications may be suggested to one skilled in the art. It is intended that the present disclosure encompasses such changes and modifications as falling in the scope of claims.

## Claims

1. A device for skin treatment comprising:
an image capturing unit (12) configured to capture an image frame of a skin area to be treated;
a motion/position sensor (14) configured to sense a displacement of the device on the skin area, and
a control means (13) configured to:
process the captured image frame during a processing period;
receive, from the motion sensor (14), a displacement of the device during the processing period; and
determine whether to perform treatment based on the received displacement.

2. The device of claim 1, wherein the control means (13) is further configured to enable a treatment source when the sensed displacement is zero and/or disable the treatment source when the sensed displacement is non-zero.

3. The device of claim 2, further comprising the treatment source (15) for performing skin treatment.

4. The device of any of the preceding claims, wherein the control means (13) is further configured to enable a treatment source (15) upon determining that the imaged treatment area is suitable for skin treatment.

5. The device of any of claims 1-4, further comprising a user interface unit (16), and wherein the control means (13) is configured to control the image capturing unit (12) to capture the image frame, upon detecting a user input to the user interface unit (16).

6. The device of any of the preceding claims, wherein the motion sensor (14) is further configured to detect an initial position of the device with respect to the skin area, and the control means (13) is configured to control the image capturing unit (12) to capture the image frame based on the detection.

7. The device of any of the preceding claims, wherein the motion sensor (14) comprises a skin contact sensor.

8. The device of claim 7, wherein the control means (13) is further configured to control the image capturing unit (12) to capture the image frame when the skin contact sensor (17) detects an initial contact of a treatment head (11) with the skin area.

9. The device of any of claims 7-8, wherein the control means (13) determines whether to perform treatment based on whether contact remains established with the imaged skin area during the processing period.

10. The device of any of the preceding claims, wherein the control means (13) is further configured to control the image capturing unit (12) to capture a new image frame during the processing period based on the received displacement.

11. The device of any of the preceding claims, wherein the processing period is 10-1000 milliseconds, preferably 100-500 milliseconds, more preferably 20-50 milliseconds.

12. The device of any of the preceding claims 3-11, wherein the treatment source is a light-based source, such as a light emitting diode, a laser or a halogen lamp.

13. A method for determining whether to perform skin treatment by a device, the method comprising:
capturing an image frame of a skin area to be treated;
sensing a displacement of the device during a processing period of the captured image frame;
determining whether to perform the skin treatment based on the sensed displacement.

14. The method of claim 13, further comprising: enabling the skin treatment when the sensed displacement is zero and disabling the skin treatment when the sensed displacement is non-zero.

15. A computer program product comprising a computer readable medium having computer readable code embodied therein, the computer readable code being configured such that, on execution by a suitable computer or control means, the computer or control means is caused to perform the method of any of claims 13-14.
